# EUROPEAN PATENT SPECIFICATION

(11) **EP 2 248 470 B1**
(45) Date of publication and mention of the grant of the patent: **10.10.2018**
(21) Application number: 09715876.0
(22) Date of filing: 25.02.2009
(51) Int. Cl.: A61B 17/00, A61M 37/00

(54) **APPLICATOR**
APPLIKATOR
APPLICATEUR

(30) Priority: 29.02.2008 JP 2008050371; 30.06.2008 JP 2008171828
(43) Date of publication of application: 10.11.2010
(73) Proprietor: Terumo Kabushiki Kaisha, Tokyo 151-0072 (JP)
(72) Inventor: HAYAKAWA, Koichi, Kanagawa 259-0151 (JP)
(74) Representative: Dossmann, Gérard
(86) International application number: PCT/JP2009/053320
(87) International publication number: WO 2009/107619

(56) References cited:
- WO-A1-2005/042079
- JP-A- 5 168 714
- JP-A- 8 281 152
- JP-A- 2001 269 348
- JP-A- 2006 087 609
- JP-T- 2001 522 270
- JP-T- 2002 513 611
- US-A- 5 364 405
- US-A1- 2006 265 035

## Description

### Technical Field

The present invention relates to an applicator. In particular, the present invention relates to applicators according to the preamble of claim 1 or the preamble of claim 7, such as they are for example known from US 5 364 405 A and WO 2005/042079 A1.

### Background Art

Hitherto, there has been known a method of mixing two or more types of liquids to inject the same to an affected part or the like, thereby forming an anti-adhesive agent, a living body tissue adhesive or the like, and an applicator for the same has been developed.

This applicator is based on a configuration in which components which are coagulated when being mixed, for example, a solution containing thrombin and a solution containing fibrinogen are sent up to the vicinity of the affected part in the state of being distinguished from each other and are applied to the affected part while being mixed.

As an applicator of the related art, there is an applicator which has two syringes respectively containing different types of liquids, and a nozzle that mixes the liquids (hereinafter, referred to as "mixed liquid") from each syringe to expel the mixed liquid (for example, see Patent Citation 1). The applicator described in Patent Citation 1 is configured so that the nozzle is connected with a gas supply source for supplying an aseptic gas to expel liquid together with the aseptic gas.

For example, in a case where the applicator as described in Patent Citation 1 is used in a laparoscopic surgery, a nozzle of the applicator is inserted into an abdominal cavity via a trochal tube which was detained (fixed) in an abdominal wall in advance. Since a direction toward which the nozzle faces is the same as an inserting direction when the nozzle is inserted into the living body, the mixed liquid cannot be expelled only toward this direction, that is, only in one direction. Thus, in the applicator described in Patent Citation 1, the mixed liquid cannot be applied to only a narrow range in the abdominal cavity, that is, only a target part present in the one direction (the direction toward which the nozzle faces), and thus the application of the mixed liquid over a wide range was impossible.

In the laparoscopic surgery, the aseptic gas is supplied into the abdominal cavity via the trochal tube. As a result, a gas abdominal pressure within the abdominal cavity rises, so that the abdominal cavity is expanded. The applicator described in Patent Citation 1 is configured so that the mixed liquid together with the aseptic gas is expelled from the nozzle as described above. Thus, the gas abdominal pressure within the abdominal cavity further rises due to the expelled aseptic gas, whereby the abdominal cavity is excessively expanded.

Patent Citation 1: JP-A-2002-282368

### Disclosure of Invention

An object of the present invention is to provide an applicator which can easily and reliably apply liquid or powder over a wide range and can suppress or prevent a rise in body pressure within a body cavity due to the expelled gas.

In order to carry out the above-mentioned object, the present invention provides an applicator according to claim 1. The claims dependent therefrom provide advantageous embodiments.

As a result, the liquid can be easily and reliably applied over the wide range, whereby it is possible to suppress or prevent a rise in body pressure within the body cavity due to the expelled gas.

In the applicator of the present invention, it is desirable that the outer tube has a front end opening portion in which a front end thereof is opened to communicate with the gap, and the front end opening portion functions as an inlet through which the gas within the body cavity flows in the gap.

As a result, the gas within the body cavity can easily flow in the gap.

In the applicator of the present invention, it is desirable that the outer tube has at least one side hole which is formed on the wall portion and communicates with the gap, and the side hole functions as an outlet through which the gas passed through the gap flows out.

As a result, the gas within the gap can reliably be discharged.

In the applicator of the present invention, it is desirable that the outer tube has a cover portion for covering the side hole from an outer peripheral side of the outer tube.

As a result, when a proximal end portion of the outer tube is grasped with fingers to perform a movement operation, it is possible to reliably prevent the side hole from being blocked by the fingers, whereby the gas passed through the gap can further reliably be discharged via the side hole.

In the applicator of the present invention, it is desirable that the outer tube has at least one side hole which is formed on the wall portion and communicates with the gap, and the side hole functions as an inlet through which the gas within the body cavity flows in the gap.

As a result, the gas within the body cavity can easily flow in the gap.

In the applicator of the present invention, as it has a positioning means for positioning the outer tube with respect to the longitudinal direction of the nozzle, as a result, it is possible to maintain a direction of the nozzle head relative to the axis of the nozzle main body.

In the applicator of the present invention, as the positioning means includes an elastic body which is fixed with respect to one surface of an outer peripheral surface of the nozzle main body and an inner peripheral surface of the outer tube in the middle of the gap and comes in contact with the other surface thereof, as a result, it is possible to reliably maintain a direction of the nozzle head relative to the axis of the nozzle main body.

In the applicator of the present invention, it is desirable that the elastic body seals the gap therein, and in the outer tube, a side hole communicating with the gap is formed on a portion of a proximal end side further than a portion where the elastic body is installed.

As a result, the gas enters the gap from the side hole, so the gas can reliably be discharged.

In the applicator of the present invention, it is desirable that the elastic body has a function of restricting a gap distance of the gap.

As a result, it is possible to reliably prevent that the inner peripheral surface of the outer tube comes in contact with the outer peripheral surface of the nozzle main body, so that the middle of the gap is blocked, whereby the gas can reliably be discharged via the gap.

In the applicator of the present invention, it is desirable that the nozzle head has a fitting portion which is fitted to the front end opening portion of the outer tube when an axis thereof coincides with an axis of the nozzle main body.

As a result, it is possible to maintain a state in which the axis of the nozzle head coincides with the axis of the nozzle main body.

In the applicator of the present invention, it is desirable that at least one groove be formed in the fitting portion along the axial direction of the nozzle head, and when the axis of the nozzle head coincides with an axis of the nozzle main body, the gas within the body cavity flows in the gap via the groove.

As a result, even when the axis of the nozzle head coincides with the axis of the nozzle main body, the groove prevents the overall front end opening portion of the outer tube from covering the nozzle head (blocking), whereby the gap communicates with the living body via the groove. As a result, the gas within the living body can reliably flow in the gap via the groove.

In the applicator of the present invention, it is desirable that the curved section be curved to the degree that the nozzle head faces the proximal end side in a natural state of not giving an external force.

As a result, it is possible to apply the liquid even to the proximal end side.

In the applicator of the present invention, it is desirable that the curved section be formed such that a shape thereof makes a U shape in the natural state, the axis of the nozzle head and the axis of the nozzle main body are parallel with each other, and the nozzle be used such that a part of the curved section is accommodated in the outer tube, a degree of the curve of the curved section is made smaller than at the time of the natural state, and the liquid and the gas are expelled from the nozzle head toward the proximal end side in this state.

As a result, it is possible to easily apply the liquid even to the proximal end side.

In the applicator of the present invention, it is desirable that the nozzle main body has a liquid flow path, which is connected to a liquid supply means for supplying the liquid and through which the liquid from the liquid supply means passes, and a gas flow path which is connected to a gas supply means for supplying the gas and through which the gas from the gas supply means passes.

As a result, it is possible to collectively expel the gas and the liquid from the nozzle head.

In order to carry out the above-mentioned object, the present invention also provides an applicator according to claim 7.

As a result, it is possible to easily and reliably apply the powder over the wide range and suppress or prevent a rise in body pressure within the body cavity due to the expelled gas. In the applicator of claim 7, as the positioning means includes a protruding portion which protrudes from one surface of the outer peripheral surface of the nozzle main body and the inner peripheral surface of the outer tube in the middle of the gap and comes in contact with the other surface thereof, as a result, it is possible to reliably maintain the direction of the nozzle head relative to the axis of the nozzle main body.

In the applicator of the present invention, it is desirable that the elastic body be intermittently disposed in plural along the outer peripheral direction of the nozzle.

As a result, a portion of the gap positioned at the front end side further than the elastic body and a portion of the gap positioned at the proximal end side further than the elastic body communicate with each other via a portion of the gap where the elastic body is not installed, that is, between the elastic bodies. Thus, the gas can enter the gap even from the front end opening portion of the sheath.

In the applicator of the present invention, it is desirable that the elastic body forms a ring shape along the outer peripheral direction of the nozzle.

As a result, it is possible to reliably perform the positioning of the outer tube relative to the longitudinal direction of the nozzle.

In the applicator of the present invention, it is desirable that the applicator has an adjusting means for adjusting the discharging amount of the gas.

As a result, for example, when the pressure in the living body is reduced, the discharging amount of the gas can be increased.

In the applicator of the present invention, it is desirable that the adjusting means includes a taper surface, which is formed on the proximal end outer peripheral portion of the nozzle main body and has an outer diameter gradually increasing toward the proximal end direction, and an inner peripheral surface of the outer tube, and the adjusting means is operated by moving the outer tube, so that the taper surface and the inner peripheral surface of the outer tube approach and are separated from each other, whereby the gap distance of the gap changes.

As a result, for example, when it is desired to reduce the pressure within the living body, the discharging amount of the gas can easily be increased.

In the applicator of the present invention, it is desirable that the nozzle be used in the state of being inserted into the trochal tube.

As a result, by protruding the nozzle head of the nozzle from the trochal tube, the nozzle head can easily be inserted into the living body.

In the applicator of the present invention, it is desirable that the trochal tube has a tubular body, a hub provided on the proximal end portion of the tubular body, and a valve main body that is provided on the hub and into which the nozzle can be inserted.

In a case where the gas is supplied into the living body via the trochal tube, even in the state in which the nozzle is inserted into the trochal tube, it is possible to prevent the gas from flowing out of the trochal tube, whereby the gas is effectively and reliably supplied into the living body.

In the applicator of the present invention, it is desirable that the applicator has a restriction means for restricting the movement limitation of the outer tube with respect to the trochal tube in the front end direction.

As a result, it is possible to prevent the proximal end portion of the outer tube from carelessly entering the trochal tube.

In the applicator of the present invention, it is desirable that the trochal tube has a tubular body and a hub provided on the proximal end portion of the tubular body, and the restriction means includes a protruding portion which protrudes from the proximal end outer peripheral portion of the outer tube and comes in contact with the proximal end portion of the hub.

As a result, it is possible to prevent the proximal end portion of the outer tube from carelessly entering the trochal tube.

### Brief Description of Drawings

Fig. 1 is a schematic partial longitudinal sectional view showing an example of a use state of an applicator (a first embodiment) of the present invention.
Fig. 2 is an enlarged partial longitudinal sectional view of the applicator shown in Fig. 1.
Fig. 3 is an enlarged partial longitudinal sectional view of the applicator shown in Fig. 1.
Fig. 4 is a longitudinal sectional view of a portion of a front end side of a nozzle in the applicator in Fig. 1.
Fig. 5 is a sectional view taken from line A-A in Fig. 2.
Fig. 6 is a transverse sectional view of a nozzle and an outer tube in an applicator (a second embodiment) of the present invention.
Fig. 7 is an enlarged partial longitudinal sectional view of an applicator (a third embodiment) of the present invention.
Fig. 8 is an enlarged partial longitudinal sectional view of an applicator (the third embodiment) of the present invention.
Fig. 9 is an enlarged partial longitudinal sectional view of a portion of a front end side of an applicator (a fourth embodiment) of the present invention.
Fig. 10 is an enlarged partial longitudinal sectional view of an applicator (a fifth embodiment) of the present invention.
Fig. 11 is an enlarged partial longitudinal sectional view of an applicator (the fifth embodiment) of the present invention.
Fig. 12 is a perspective view of a portion of a front end side of the applicator shown in Fig. 10.
Fig. 13 is a perspective view of a portion of a front end side of the applicator shown in Fig. 11.
Fig. 14 is an enlarged partial longitudinal sectional view of an applicator (a sixth embodiment) of the present invention.
Fig. 15 is an enlarged partial longitudinal sectional view of the applicator (the sixth embodiment) of the present invention.
Fig. 16 is an enlarged partial longitudinal sectional view of the applicator (the sixth embodiment) of the present invention.
Fig. 17 is an enlarged partial longitudinal sectional view of an applicator (a seventh embodiment) of the present invention.
Fig. 18 is a sectional view taken from line B-B in Fig. 17.

### Best Mode for Carrying Out the Invention

Hereinafter, an applicator of the present invention will be described in detail based on preferable embodiments which are shown in the attached drawings.

### First Embodiment

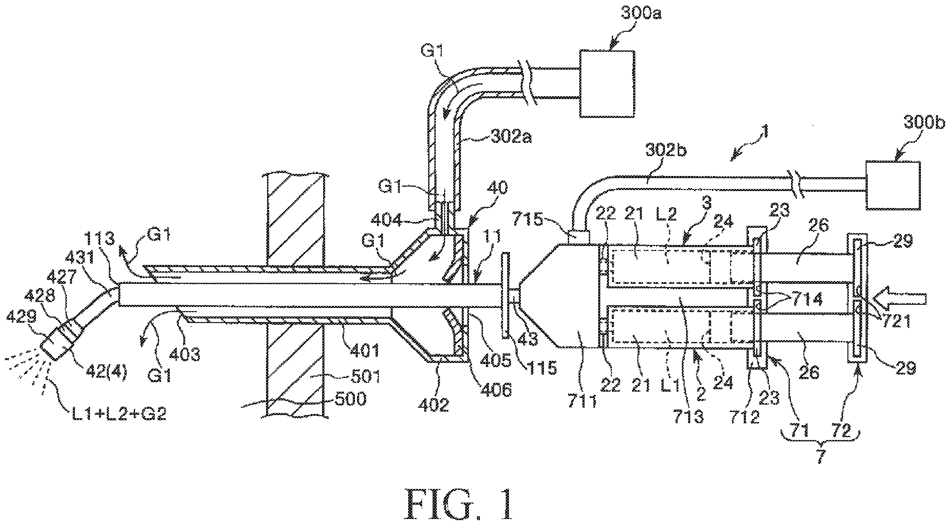

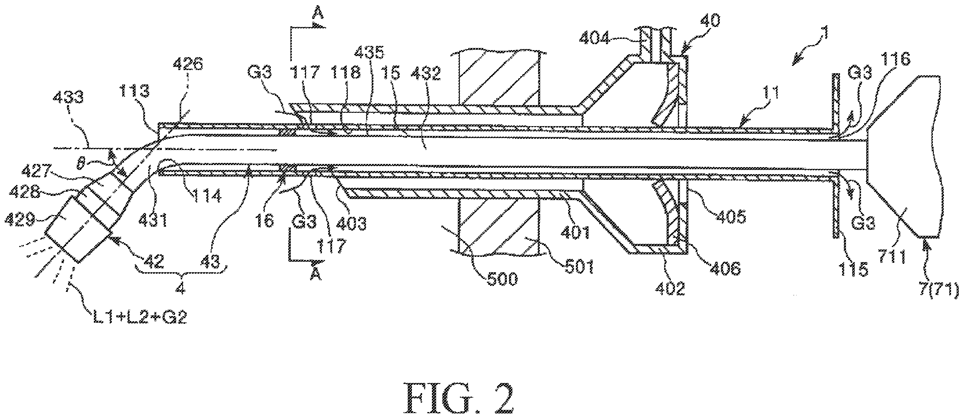

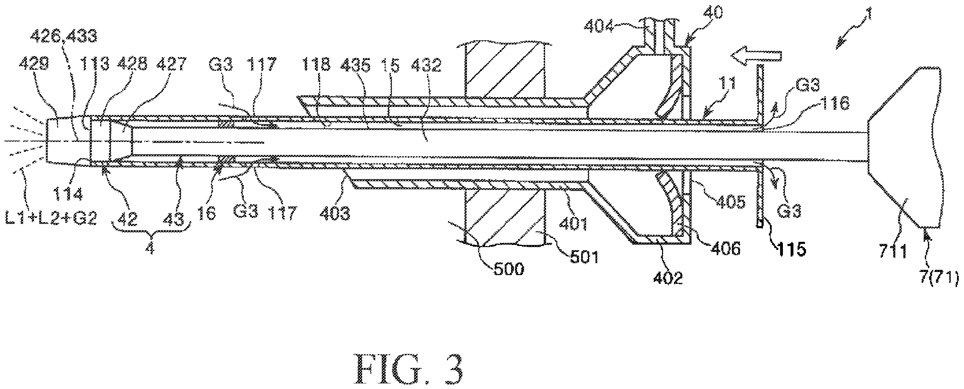

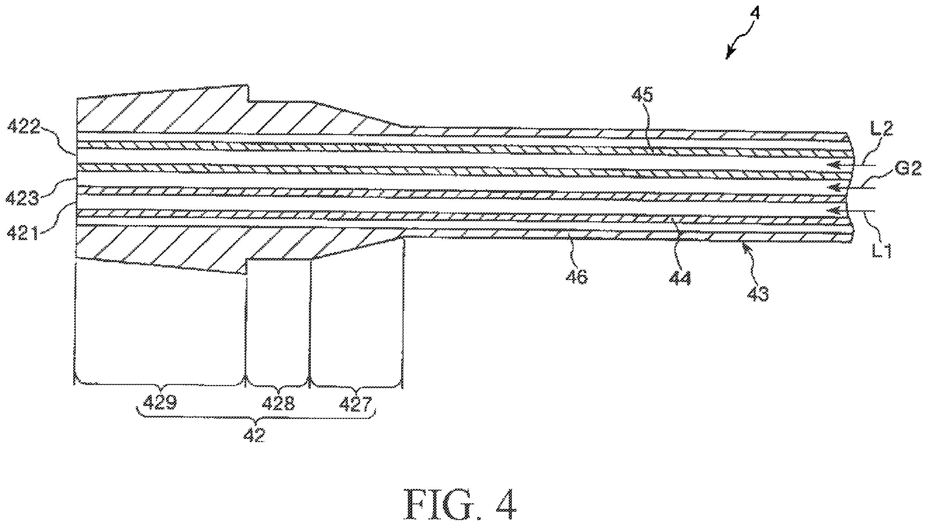

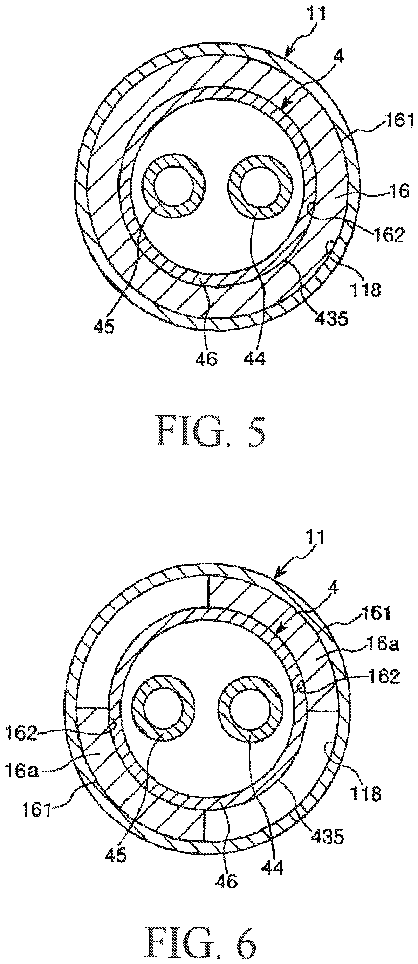

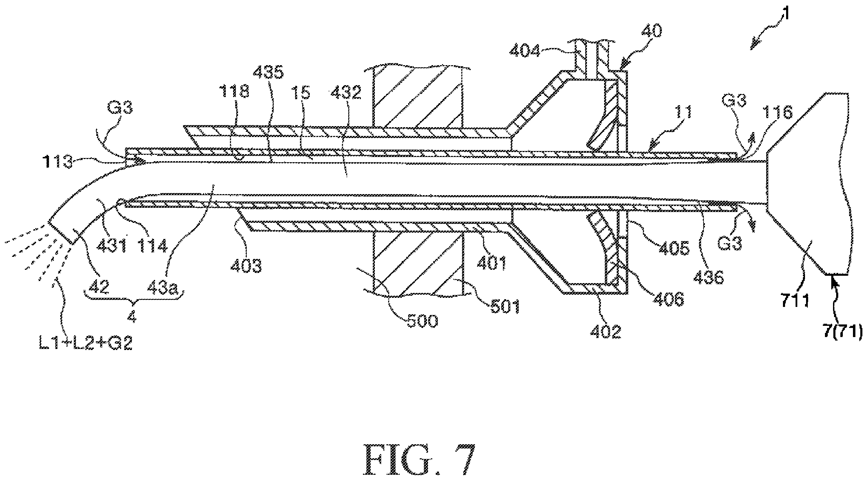

Fig. 1 is a schematic partial longitudinal sectional view showing an example of a use state of an applicator (a first embodiment) of the present invention. Figs. 2 and 3 are enlarged partial longitudinal sectional views of the applicator shown in Fig. 1. Fig.4 is a longitudinal sectional view of a portion of a front end side of a nozzle in the applicator in Fig. 1. Fig. 5 is a sectional view taken from line A-A in Fig. 2. Hereinafter, for convenience of description, a left side in Figs. 1 to 4 (similarly, as for Figs. 7 to 17) is called "a front end," and a right side thereof is called "a proximal end (a rear end)".

As shown in Fig. 1, applicator 1 is inserted into abdominal cavity 500, for example, during laparoscopic surgery to apply a mixture of two types of liquids with different liquid compositions (first liquid L1 and second liquid L2) to organs, abdominal wall 501 or the like, while mixing the liquids. The insertion of applicator 1 into abdominal cavity 500 is performed via trochal tube 40 which was detained in abdominal wall 501 in advance. Specifically, nozzle 4 of applicator 1 is inserted into trochal tube 40 to protrude nozzle head 42 of nozzle 4 from trochal tube 40, whereby applicator 1 (nozzle head 42) can be inserted into abdominal cavity 500.

Before describing applicator 1, trochal tube 40 will be described.

As shown in Fig. 1, trochal tube 40 has main body portion (tubular body) 401 forming a tubular shape, and hub 402 provided on the proximal end portion of main body portion 401.

Main body portion 401 is that which the front end and the proximal end thereof are opened. Front end opening portion 403 of main body portion 401 is sloped with respect to an axis of main body portion 401. As a result, the front end portion of trochal tube 40 becomes a shape which makes it easier to insert trochal tube 40 from the front end side into abdominal cavity 500. Thus, it is possible to easily perform the insertion operation of trochal tube 40 into abdominal cavity 500.

Hub 402 is a part, which has an inner diameter and outer diameter larger than those of main body portion 401, and communicates with main body portion 401.

On an outer peripheral portion of hub 402, gas supply port 404 is protruded. Gas supply port 404 is connected with gas cylinder (a gas supply means) 300a via tube 302a. Gas (aseptic gas) G1 supplied from gas cylinder 300a sequentially passes through tube 302a, gas supply port 404, hub 402 and main body portion 401 and is supplied into abdominal cavity 500 (see Fig. 1). Due to the supply of gas G1, a gas abdominal pressure in abdominal cavity 500 rises further than an atmospheric pressure by 8 to 12 mmHg, whereby abdominal cavity 500 is expanded. As a result, abdominal cavity 500 becomes a size which is sufficient for performing the laparoscopic surgery. In addition, gas cylinder 300a has the same configuration as gas cylinder 300b as described later, and the description thereof will be omitted therein.

On proximal end opening portion 405 of hub 402, duckbill valve (a valve main body) 406 is installed to cover proximal end opening portion 405. Duckbill valve 406 is closed in the state in which applicator 1 is not inserted into trochal tube 40, and is opened when applicator 1 is inserted into trochal tube 40. Duckbill valve 406 can prevent gas G1 from flowing out of proximal end opening portion 405 even in the state in which applicator 1 is inserted. Thus, gas G1 is effectively and reliably supplied into abdominal cavity 500.

Main body portion 401 and hub 402 may be formed integrally, and may be configured separately, so that the separated bodies are connected and fixed to each other.

As described above, applicator 1 applies the two types of liquids with different liquid compositions (first liquid L1 and second liquid L2) while mixing them (see Figs. 1 to 3) . Applicator 1 is used by loading first syringe (a liquid supply means) 2 for containing first liquid L1 and second syringe (the liquid supply means) 3 for containing second liquid L2 therein. Since first syringe 2 and second syringe 3 have substantially the same configuration, first syringe 2 will be described representatively.

First syringe 2 includes outer case 21, gasket 24 capable of sliding in outer case 21, and pusher 26 for moving and operating casket 24 along a longitudinal direction (an axial direction) of outer case 21. Gasket 24 is connected with a front end of pusher 26.

Outer case 21 is formed by cylindrical member with a bottom, and, a reduced diameter portion (a mouth portion) 22, which is reduced in diameter with respect to the body portion of outer case 21, is integrally protruded and formed on a center portion of a front end side bottom portion.

Flange 23 is integrally formed on a rear end outer periphery of outer case 21. Gradations which indicate liquid amounts are added to the outer peripheral surface of outer case 21.

As construction materials of outer case 21, although it is not particularly limited, for example, various resins as described in regard to trochal tube 40 can be used. In addition, it is desirable the construction materials of outer case 21 be substantially transparent so as to secure visibility of the inner part.

Gasket 24 formed of an elastic material is accommodated into outer case 21. The outer peripheral surface of Gasket 24 comes into close contact with the inner peripheral surface of outer case 21 while sliding, whereby first liquid L1 can be pushed toward mouth portion 22 while reliably maintaining a liquid-tight property in outer case 21.

The movement of Gasket 24 is performed by moving and operating pusher 26. Pusher 26 is a long member, and has disk-shaped flange 29 formed on the end side thereof.

Before first syringe 2 is loaded to applicator 1, first liquid L1 is charged in a space (a liquid containing space) surrounded by outer case 21 and gasket 24. In second syringe 3, second liquid L2 is charged in a space (a liquid containing space) surrounded by outer case 21 and gasket 24.

First liquid L1 charged in first syringe 2 and second liquid L2 charged in second syringe 3 are different from each other in composition (ingredients).

In the present invention, first liquid L1 and second liquid L2 are suitably selected corresponding to a use, a using purpose, an index case of applicator 1 or the like. For example, when they are used for administering a living body tissue adhesive, one of first liquid L1 and second liquid L2 can be a liquid containing thrombin, and the other thereof can be a liquid containing fibrinogen.

When first liquid L1 and second liquid L2 are used for administering the anti-adhesive agent, one of them can be a liquid containing carboxylmethyl dextrin modified with succinimidyl radical, and the other of them can be a liquid containing disodium dihydrogen phosphate which is a pH regulator.

First liquid L1 and second liquid L2 with this combination spoil, that is, gel (solidify) when they are mixed with each other. Due to gel, for example, the mixture (hereinafter, often called "mixture") of first liquid L1 and second liquid L2 can reliably remain in the applied living body tissue (a target part). In addition, since the mixture reliably remains in the target part, a function as the living body tissue adhesive or the anti-adhesive agent can reliably be exhibited in the target part.

In addition, it is needless to say that the types or the combinations of first liquid L1 and second liquid L2 are not limited to the above description.

First syringe 2 and second syringe 3 with the configuration are connected to nozzle 4 as described later and press and operate the respective pushers 26, whereby they can easily and reliably supply first liquid L1 to a first flow path 44 of nozzle 4 and second liquid L2 to a second flow path 45. The pressing operations of the respective pushers 26 are manually performed by an operator of applicator 1. For this reason, the operator can perform the application of the mixture at his own arbitrary timing.

Applicator 1 expels first liquid L1 and second liquid L2 together with gas G2 (see Figs. 1 to 3). Due to gas G2, the mixture is atomized, whereby the mixture can uniformly be applied to the target part. Gas G2 is supplied by a gas cylinder 300b. Gas cylinder 300b is connected with the nozzle via a tube 302b.

Gas cylinder 300b has the compressed aseptic gas G2 (hereinafter, merely called "gas G2") charged in an inner space thereof and can supply gas G2 flowing at a high speed to applicator 1 (nozzle 4). In the middle of gas cylinder 300b or tube 302b, an openable and closable valve (not shown), which controls the supply/the supply stop of gas G with respect to applicator 1, is installed. When applying the mixture, the valve is in the open state.

As shown in Figs. 1 to 3, applicator 1 includes applicator main body 7, nozzle 4 provided on the front end side of applicator main body 7, and the sheath (outer tube) 11 through which nozzle 4 is inserted.

As shown in Fig. 1, applicator main body 7 includes syringe holding portion 71, which holds outer case 21 of first syringe 2 and outer case 21 of second syringe 3, and flange connecting portion 72 which connects flange 29 of pusher 26 of first syringe 2 with flange 29 of pusher 26 of second syringe 3.

Syringe holding portion 71 fixes first syringe 2 (outer case 21) and second syringe 3 (outer case 21) in parallel. Syringe holding portion 71 has a fitting portion 711 to which mouth portion 22 of each outer case 21 is fitted, inserting portion 712 which is situated at the proximal end side further than fitting portion 711 and into which an edge portion of flange 23 of each outer case 21 is inserted, and a connecting portion 713 for connecting fitting portion 711 with inserting portion 712.

When mouth portion 22 of each outer case 21 is fitted to fitting portion 711, mouth portion 22 of first syringe 2 is connected to first flow path 44 of nozzle 4, and mouth portion 22 of second syringe 3 is connected to second flow path 45. As a result, it is possible to supply first liquid L1 to first flow path 44 and second liquid L2 to second flow path 45 (see Fig. 4).

On the outer peripheral portion of fitting portion 711, connection portion 715 is protruded and formed to which an end portion of tube 302b, through which gas G2 from gas cylinder 300b passes, is connected. When tube 302b is connected to connection portion 715, tube 302b is connected to third flow path 46 of nozzle 4. As a result, it is possible to supply gas G2 to third flow path 46 (see Fig. 4).

On inserting portion 712, grooves 714 into which the edge portion of flange 23 of outer case 21 is inserted are formed.

In the syringe holding portion 71, mouth portions 22 of each outer case 21 are fitted to fitting portion 711, and flange 23 of outer case 21 is inserted into inserting portion 712 (the grooves 714), whereby each outer case 21 can reliably be maintained.

Flange connecting portion 72 is a plate-shaped member that connects flange 29 of pusher 26 of first syringe 2 with flange 29 of pusher 26 of second syringe 3. On flange connecting portion 72, grooves 721 into which the edge portions of the flanges 29 of each pusher 26 are inserted are formed. By pressing flange connecting portion 72 toward the front end direction, each pusher 26 can collectively be moved toward the front end direction. In this manner, when applicator 1 is used, that is, when the mixture is applied to the target part such as an affected part, flange connecting portion 72 functions as an operating portion which is pressed and operated by the user.

As construction materials of syringe holding portion 71 and flange connecting portion 72, for example, various resins materials can be used.

Nozzle 4 is installed on the front end side of applicator main body 7. Nozzle 4 expels gas G2 together with first liquid L1 and second liquid L2 (mixture).

As shown in Figs. 2 to 4, nozzle 4 has longer length shaped nozzle main body 43 and a nozzle head 42 provided on the front end of nozzle main body 43.

As shown in Fig. 4, nozzle main body 43 has first flow path 44 through which first liquid L1 from first syringe 2 passes, second flow path 45 through which second liquid L2 from second syringe 3 passes, and third flow path 46 through which gas G2 from gas cylinder 300b passes.

First flow path 44 and second flow path 45 are respectively formed of inner tubes. In the respective inner tubes, the front ends thereof are extended up to the front end surface of nozzle head 42 and are opened at the front end surfaces thereof. In nozzle head 42, the opening portion of inner tube constituting first flow path 44 functions as first discharging port 421 for discharging first liquid L1, and the opening portion of inner tube constituting second flow path 45 functions as second discharging port 422 for discharging second liquid L2. Inner tube constituting first flow path 44 extends up to a position where the proximal end portion thereof is connected to mouth portion 22 of first syringe 2. Similarly, inner tube constituting second flow path 45 extends up to a position where the proximal end portion thereof is connected to mouth portion 22 of second syringe 3.

Third flow path 46 is constituted by an outer tube, which is situated on the outer periphery side of the inner tube which respectively constitutes first flow path 44 and second flow path 45, that is, through which each inner tube is inserted. The outer tube is configured so that the front end thereof extends up to the front end surface of nozzle head 42 and is opened at the front end surface. In nozzle head 42, the opening portion of the outer tube functions as third discharging port 423 for expelling gas G2. In addition, the outer tube extends up to a position where the proximal end portion thereof is connected to tube 302b via connection portion 715 of applicator main body 7.

The respective inner tube and the outer tube are in the positional relationship as described above. Thus, on the outer periphery sides of first discharging port 421 and second discharging port 422, that is, in order to surround first discharging port 421 and second discharging port 422, a third discharging port 423 is disposed. As a result, first liquid L1 discharged from first discharging port 421 and second liquid L2 discharged from second discharging port 422 are mixed with gas G2 discharged from third discharging port 423 at a high speed. At this time, first liquid L1 and second liquid L2 are respectively atomized and expelled. As a result, first liquid L1 and second liquid L2 are reliably mixed with each other and are applied to the affected part.

As shown in Figs. 1 and 2, nozzle main body 43 has a curved section 431, which is curved or bent and has flexibility, at the front end portion thereof. In the present embodiment, curved section 431 is curved or bent so that the front end thereof faces obliquely downward. Due to curved section 431, an axis 426 of nozzle head 42 slopes with respect to an axis (strictly, an axis of portion 432 of the proximal end side further than curved section 431 of nozzle main body 43) 433 of nozzle main body 43 (see Fig. 2).

When curved section 431 is curved or bent without being restricted by sheath 11 described later, a slope angle θ of axis 426 of nozzle head 42 with respect to axis 433 of nozzle main body 43 is preferably about 30 to 90°, and more preferably about 45 to 70°.

Curved section 431 of nozzle main body 43 is formed of a soft material, an elastic material or the like. In addition, portion 432 of the proximal end side further than curved section 431 of nozzle main body 43 may be formed of a rigid material, a soft material, or may be formed of an elastic material or the like and may have flexibility.

Curved section 431 and portion 432 on the proximal end side further than curved section 431 of nozzle main body 43 may be configured so that they are formed of separated members and are fixed by bonding, melting or the like, and may be configured by being integrally formed.

The construction material of nozzle 4 (nozzle main body 43) includes, for example, various thermoplastic elastomers such as polyurethane base, polyester base, polyamide base, olefin base, and styrene base. Portion 432 on the proximal end side further than curved section 431 of nozzle main body 43 can use any of those materials. Curved section 431 can use the soft material or the elastic material among them.

As described above, nozzle head 42 is that which first liquid L1 is discharged from first discharging port 421, second liquid L2 is discharged from second discharging port 422, and gas G2 is expelled from third discharging port 423 while mixing the liquids.

Nozzle head 42 has a cylindrical outer shape, and has first taper portion 427 with a gradually increasing outer diameter toward the front end direction, outer diameter constant portion 428 with a constant outer diameter along a longitudinal direction (axis 426), and second taper portion 429 with a gradually decreasing outer diameter toward the front end direction, which are disposed from the proximal end side in this order.

First taper portion 427 is formed, so that when nozzle 4 changes from the state shown in Fig. 2 to the state shown in Fig. 3, inner edge portion 114 of front end opening portion 113 of sheath 11 can smoothly move along first taper portion 427. As a result, nozzle head 42 can be reliably introduced into sheath 11. In addition, it is desirable that edge portion 114 be spherical. As a result, edge portion 114 can further smoothly move along first taper portion 427.

Second taper portion 429 is situated on the front end side further than first taper portion 427. Second taper portion 429 is formed, so that, for example, when applicator 1 is inserted into trochal tube 40 in the state shown in Fig. 3, second taper portion 429 can smoothly pass through duckbill valve 406 of trochal tube 40. As a result, the inserting operation of applicator 1 into trochal tube 40 can easily be performed. Furthermore, the outer diameter of the proximal end of second taper portion 429 is larger than the inner diameter of sheath 11. As a result, it is possible to prevent nozzle head 42 from entering sheath 11, namely, prevent the front end of nozzle head 42 from moving toward the proximal end side further than the front end of sheath 11.

Outer diameter constant portion 428 is situated between first taper portion 427 and second taper portion 429. The outer diameter of outer diameter constant portion 428 is the same as the outer diameter (maximum outer diameter) of the front end of first taper portion 427, and is equal to or slightly larger than the inner diameter of sheath 11. As a result, as shown in Fig. 3, when axis 426 of nozzle head 42 coincides with axis 433 of nozzle main body 43, outer diameter constant portion 428 (also including the front end of first taper portion 427) enters and is fitted to front end opening portion 113 of sheath 11. Thus, it is possible to maintain the state in which axis 426 of nozzle head 42 coincides with axis 433 of nozzle main body 43. The mixture can be applied to the target part within abdominal cavity 500 in the maintained state. In applicator 1, outer diameter constant portion 428 and the front end of first taper portion 427 can be called "a fitting portion" which is fitted to front end opening portion 113 of sheath 11.

As shown in Fig. 2 (similar to Figs. 1 and 3), applicator 1 has sheath 11 with a function as a form restricting member that restricts the shape of curved section 431 of nozzle main body 43. Sheath 11 is formed by a tubular body with a longer length shape in which the front end and the proximal end thereof are respectively opened, and nozzle 4 (nozzle main body 43) is inserted into sheath 11. In the present embodiment, sheath 11 is adapted to be inserted from the portion (outer diameter constant portion 428 of nozzle head 42) on the front end side further than curved section 431 up to the vicinity of the proximal end portion of nozzle main body 43. Sheath 11 is adapted to be movable along the longitudinal direction of nozzle main body 43 with respect to nozzle main body 43.

It is desirable that sheath 11 be formed of a rigid material and have necessary and sufficient stiffness so that it can restrict the shape of curved section 431 when covering a part or all of curved section 431, as well as having a low sliding property.

The construction material of sheath 11 includes a polyolefin resin such as polyethylene or polypropylene, a fluorine resin such as polytetra fluoroethylene, and a rigid resin such as polycarbonate, polyethylene terephthalate or polyamide.

As described above, sheath 11 can be moved and operated along the longitudinal direction of nozzle main body 43. Due to the movement operation, curved section 431 is inserted into sheath 11, and the protrusion length of curved section 431 from the front end of sheath 11 is adjusted, whereby the angle of curved section 431 can be changed (see Figs. 2 and 3). As a result, slope angle θ (the direction of nozzle head 42) of axis 426 of nozzle head 42 with respect to axis 433 of nozzle main body 43 can be adjusted. In other words, sheath 11 can move between a first position (slope angle θ=0°) where curved section 431 is restricted by sheath 11 and becomes a linear shape and a direction of axis 426 of nozzle head 42 coincides with a direction of axis 433 of nozzle main body 43, as shown in Fig. 3, and a second position (slope angle θ is the maximum slope angle) where curved section 431 enters the curved or bent state without being restricted by sheath 11 and axis 433 of nozzle main body 43 slopes with respect to axis 426 of nozzle head 42, as shown in Fig. 2. As a result, by moving sheath 11 to a predetermined position between the first position and the second position, slope angle θ can freely be adjusted within the range from 0° to the maximum slope angle.

As described above, it is possible to spray the mixture from nozzle head 42 toward a plurality of places within abdominal cavity 500 (for example, the organ or abdominal wall 501), while changing slope angle θ of nozzle head 42 by moving sheath 11 to suitably adjust slope angle θ. Thus, applicator 1 can easily and reliably apply the mixture in abdominal cavity 500 over the wide range. In addition, in applicator 1, the mixture can be applied even to the rear side of abdominal wall 501 which faces the organ positioned in the inserting direction of applicator 1 into the living body, by suitably setting (e.g., a "U" shape) a degree (slope angle θ) of the curve of curved section 431 in the natural state in which the external force is not given.

Sheath 11 has plate-shaped flange 115 protruded and formed at the proximal end outer peripheral portion thereof. Sheath 11 can be moved and operated by grasping flange 115 and moving the same along the longitudinal direction of sheath 11. In this manner, flange 115 functions as an operating portion when moving sheath 11.

As described above, applicator 1 is used in the state of being inserted into trochal tube 40. In this state, when applicator 1 is gradually pressed toward the front end direction, flange 115 of sheath 11 comes into contact with proximal end opening portion 405 of hub 402. As a result, the movement limitation of sheath 11 with respect to trochal tube 40 in the front end direction can be restricted, which makes it possible to prevent the proximal end portion of sheath 11 from unintentionally entering trochal tube 40, that is, to prevent the proximal end portion of sheath 11 from moving to the front end side further than proximal end opening portion 405 of trochal tube 40. As a result, the proximal end portion of sheath 11 can reliably protrude from proximal end opening portion 405 of trochal tube 40 to grasp the protruded portion. Thus, sheath 11 can be moved and operated. In this manner, flange 115 also functions as a restriction means that restricts the movement limitation of sheath 11 with respect to trochal tube 40 in the front end direction.

In addition, an operation for adjusting slope angle θ and an operation for discharging (expelling) first liquid L1 and second liquid L2 can be performed in any order.

That is, the operation for adjusting slope angle θ and the operation for expelling first liquid L1 and second liquid L2 may concurrently be performed, and the operation for expelling first liquid L1 and second liquid L2 begins first, and then, the operation for adjusting slope angle θ may be performed. In this case, after first liquid L1 and second liquid L2 begin to be expelled, a range of performing the application can be widened by the adjustment of slope angle θ. In addition, the operation for adjusting slope angle θ is performed first, and then, the operation for expelling first liquid L1 and second liquid L2 may be performed.

Incidentally, as shown in Figs. 2 and 3, gap 15 is formed between sheath 11 and nozzle 4. Gap 15 is formed along the longitudinal direction of sheath 11, that is, from front end opening portion 113 of sheath 11 over proximal end opening portion 116.

Sheath 11 has two side holes 117 passing through the wall portion, formed on the proximal end side further than front end opening portion 113. Side holes 117 are oppositely disposed at the same position with respect to the longitudinal direction of sheath 11 via the axis of sheath 11. Each side hole 117 communicates with gap 15 and function as inlet (blow-in ports through which gas G3 is blown in) through which gas G3 within abdominal cavity 500 flows in gap 15 (see Figs. 2 and 3). The number of side holes 117 is not limited to two, but, for example, may be one or three or more.

Gap 15 functions as a discharge path for discharging gas G3 within abdominal cavity 500 from the body. Hereinafter, this will be described.

As shown in Fig. 1, gas G1 is expelled from trochal tube 40 into abdominal cavity 500, and gas G2 together with the mixture is expelled from applicator 1. As described above, due to gas G1, the gas abdominal pressure within abdominal cavity 500 rises and abdominal cavity 500 is expanded. The gas abdominal pressure within abdominal cavity 500 also rises due to gas G2, whereby abdominal cavity 500 tries to further expand. However, gas G3 (including the gases G1 and G2) within abdominal cavity 500 flows in gap 15 via each side hole 117 (see Figs. 2 and 3). Gas G3 flows down in gap 15 and is discharged from proximal end opening portion 116 (see Figs. 2 and 3). As a result, an excessive rise in gas abdominal pressure within abdominal cavity 500 can be suppressed (or prevented), which can prevent abdominal cavity 500 from trying to further expand.

As described above, applicator 1 is configured so that it can easily and reliably apply the mixture within abdominal cavity 500 over the wide range. Since the mixture is applied within abdominal cavity 500 over the wide range, the expelling amount of the mixture also increases, and the expelling amount of gas G2 also increases. When a large amount of gas G2 is expelled, the gas abdominal pressure in abdominal cavity 500 seems to be increased; however, the exhaust operation of the gap can suppress the rises. In this manner, applicator 1 is effective even in a case where the mixture is applied within abdominal cavity 500 over the wide range.

In the middle of gap 15, ring-shaped spacer 16 is installed along the outer peripheral direction of nozzle 4 (see Figs. 2, 3 and 5). In spacer 16, outer peripheral surface 161 thereof is fixed to inner peripheral surface 118 of sheath 11, and inner peripheral surface 162 thereof is in contact with outer peripheral surface 435 of nozzle main body 43 (see Fig. 5). When sheath 11 moves, inner peripheral surface 162 of spacer 16 slides on outer peripheral surface 435 of nozzle main body 43, whereby a frictional resistance is generated between outer peripheral surface 435 of nozzle main body 43 and inner peripheral surface 162 of spacer 16.

Spacer 16 with this configuration functions as a positioning means that performs the positioning of sheath 11 with respect to the longitudinal direction of nozzle 4 in the stop position, when sheath 11 is caused to move and stop. As a result, slope angle θ of nozzle head 42 can be maintained, so that the mixture can be expelled in this state.

Spacer 16 is installed between inner peripheral surface 118 of sheath 11 and outer peripheral surface 435 of nozzle main body 43 in the compressed state. As a result, spacer 16 is not easily deformed in the diameter direction thereof, whereby a function of maintaining the gap distance of gap 15 is also exhibited. As a result, inner peripheral surface 118 of sheath 11 comes in contact with outer peripheral surface 435 of nozzle main body 43, so that it is possible to reliably prevent the middle of gap 15 (the exhaust path) from being blocked, which can reliably exhaust gas G3 via gap 15.

As shown in Fig. 2, spacer 16 is disposed on the front end side further than each side hole 117. In this position, spacer 16 seals gap 15. In the state shown in Fig. 3, although nozzle head 42 blocks front end opening portion 113 of sheath 11, since gas G3 enters gap 15 from each side hole 117, gas G3 can reliably be discharged.

The construction material of spacer 16 is not particularly limited, for example, various elastic materials can be used as described regarding duckbill valve 406.

### Second Embodiment

Fig. 6 is a transverse sectional view of a nozzle and an outer tube in an applicator (a second embodiment) of the present invention.

Hereinafter, a second embodiment of an applicator of the present invention will be described with reference to the drawing, but the description will be focused on the points different from the above-mentioned embodiment, and the descriptions of the identical matters will be omitted.

The present embodiment is the same as the above-mentioned first embodiment except for a difference in arrangement state of the elastic body.

As shown in Fig. 6, spacers 16a, which function as a positioning means for positioning sheath 11 with respect to the longitudinal direction of nozzle 4, are installed around the axis of nozzle 4 (along the outer peripheral direction of nozzle 4) in plural numbers (two in the shown configuration) . Each spacer 16a has a circular shape and is disposed around the axis of nozzle 4 at equal angle distances. As a result, a portion of gap 15 on the front end side further than spacer 16a and a portion of gap 15 on the proximal end side further than spacer 16a communicate with each other via a portion of gap 15 where spacer 16a is not installed, namely, between spacers 16a. As a result, in the state in which front end opening portion 113 of sheath 11 is not sealed by nozzle head 42 (the state shown in Fig. 2), gas G3 enters gap 15 even from front end opening portion 113 other than the side holes 117. Gas G3 is discharged from the proximal end opening portion 116. In this manner, in the present embodiment, front end opening portion 113 functions as an inlet through which gas G3 within abdominal cavity 500 flows in gap 15.

Furthermore, in the present embodiment, the discharging amount of gas G3 can be adjusted.

Specifically, when sheath 11 moves in the front end direction from the state shown in Fig. 2, the outer peripheral surface of first taper portion 427 of nozzle head 42 and inner peripheral surface 118 of sheath 11 approach each other. When, from this state, contrary to the above, sheath 11 moves in the proximal end direction, the outer peripheral surface of first taper portion 427 of nozzle head 42 is separated from inner peripheral surface 118 of sheath 11. Due to the approach and the separation, the gap distance of gap 15 near front end opening portion 113 of sheath 11 changes. As a result, the inflow amount of gas G3 flowing in from front end opening portion 113 is adjusted, and consequently, the discharging amount of gas G3 can be adjusted. As a result, for example, in a case where it is desired to reduce the gas abdominal pressure in abdominal cavity 500, by operating sheath 11 so that the outer peripheral surface of first taper portion 427 of nozzle head 42 is separated from inner peripheral surface 118 of sheath 11, the discharging amount of gas G3 can be improved.

In this manner, in the present embodiment, the outer peripheral surface of first taper portion 427 of nozzle head 42 and inner peripheral surface 118 of sheath 11 form an adjusting means for adjusting the discharging amount of gas G3.

### Third Embodiment

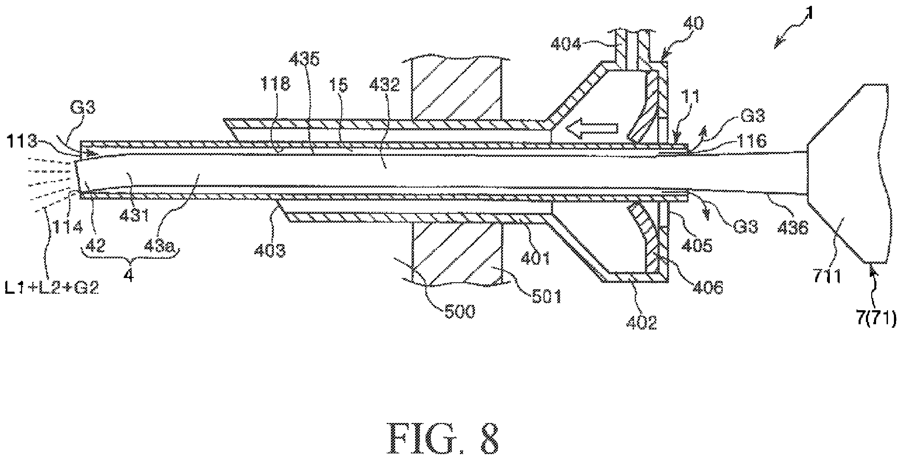

Figs. 7 and 8 are enlarged partial longitudinal sectional views of the applicator (a third embodiment) of the present invention.

Hereinafter, although the third embodiment of the applicator of the present invention will be described with reference to the drawings, the description will be focused on the points different from the above-mentioned embodiments, and the description of the same matters will be omitted.

The present embodiment is the same as the first embodiment except that a shape of a nozzle and a constitution of an outer tube differ.

As shown in Figs. 7 and 8, on a proximal end outer peripheral portion of a nozzle main body 43a, a taper surface 436, which has an outer diameter gradually increasing toward the proximal end direction, is formed.

When sheath 11 moves in the front end direction from the state shown in Fig. 7, the taper surface 436 of nozzle main body 43a is separated from inner peripheral surface 118 of sheath 11 (see Fig. 8). Contrary to the above, when sheath 11 moves in the proximal end direction from the state shown in Fig. 8, the taper surface 436 of nozzle main body 43a approaches inner peripheral surface 118 of sheath 11. Due to the approach and the separation, the gap distance of gap 15 near proximal end opening portion 116 of sheath 11 changes. As a result, the outflow amount of gas G3 flowing out of proximal end opening portion 116 is adjusted, and consequently, the discharging amount of gas G3 can be adjusted. As a result, in a case where, for example, it is desired to reduce the gas abdominal pressure in abdominal cavity 500, by operating sheath 11 so that the taper surface 436 of nozzle main body 43a is separated from inner peripheral surface 118 of sheath 11, the discharging amount of gas G3 can be improved.

In this manner, in the present embodiment, the taper surface 436 of nozzle main body 43a and inner peripheral surface 118 of sheath 11 form an adjusting means for adjusting the discharging amount of gas G3.

In the present embodiment, nozzle head 42 is configured so that a portion in which the outer diameter changes along the longitudinal direction is omitted and it becomes constant. The outer diameter of nozzle head 42 is smaller than the inner diameter of sheath 11. As a result, regardless of the position of sheath 11 in the longitudinal direction of nozzle 4, gas G3 flows in gap 15 from front end opening portion 113 of sheath 11.

In the present embodiment, on the proximal end outer peripheral portion of sheath 11, flange 115 protruding from the proximal end outer peripheral portion of sheath 11 as described in the first embodiment is omitted, but not limited thereto, flange 115 may be formed.

### Fourth Embodiment

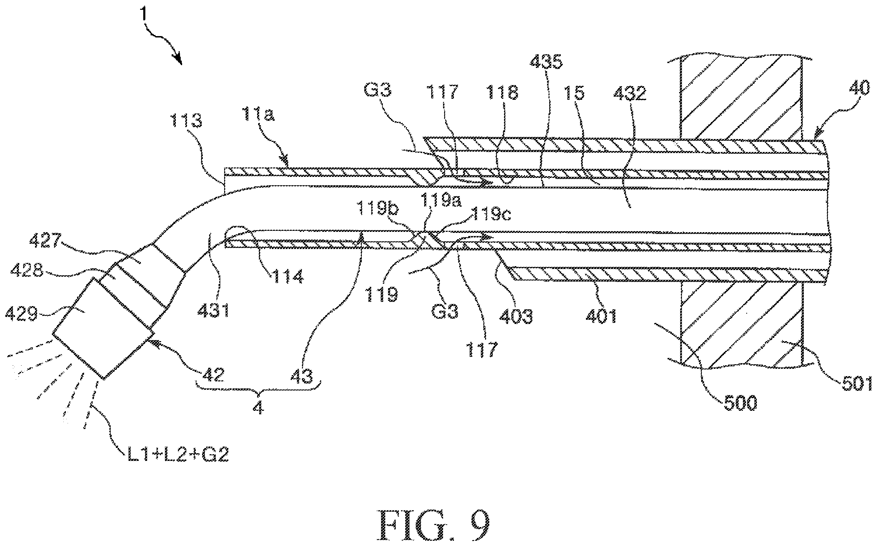

Fig. 9 is an enlarged partial longitudinal sectional view of a portion on a front end side of an applicator (a fourth embodiment) of the present invention.

Hereinafter, although a fourth embodiment of an applicator of the present embodiment will be described with reference to the drawings, the description will be focused on the points different from the above-mentioned embodiment and the description of the same matter will be omitted.

The present embodiment is the same as the above-mentioned first embodiment except that a positioning means has a different configuration.

As shown in Fig. 9, on inner peripheral surface 118 of a sheath 11a, protruding portion 119 protrudes in the vicinity of the front end of side hole 117. Protruding portion 119 forms a ring shape along the circumferential direction of inner peripheral surface 118 of sheath 11a. As a result, in protruding portion 119, the inner diameter of sheath 11a is reduced. In protruding portion 119, peak portion 119a is in contact with outer peripheral surface 435 of nozzle main body 43. As a result, when sheath 11a moves, peak portion 119a of protruding portion 119 slides on outer peripheral surface 435 of nozzle main body 43, so that the frictional resistance is generated between peak portion 119a of protruding portion 119 and outer peripheral surface 435 of nozzle main body 43. When the movement of sheath 11a stops, the positioning of sheath 11a with respect to the longitudinal direction of nozzle 4 in the stop position is performed. As a result, slope angle θ of nozzle head 42 can be maintained, whereby the mixture can be expelled in this state. In this manner, protruding portion 119 functions as a positioning means.

In protruding portion 119, peak portion 119a has a spherical shape. As a result, when sheath 11a moves, it is possible to reduce the frictional resistance generating between peak portion 119a of protruding portion 119 and outer peripheral surface 435 of nozzle main body 43, and thus the movement operation thereof can easily be performed.

Both surfaces 119b and 119c via peak portion 119a of protruding portion 119, i.e., the surfaces 119b and 119c on the front end side and the proximal end side of protruding portion 119 are respectively sloped surfaces.

### Fifth Embodiment

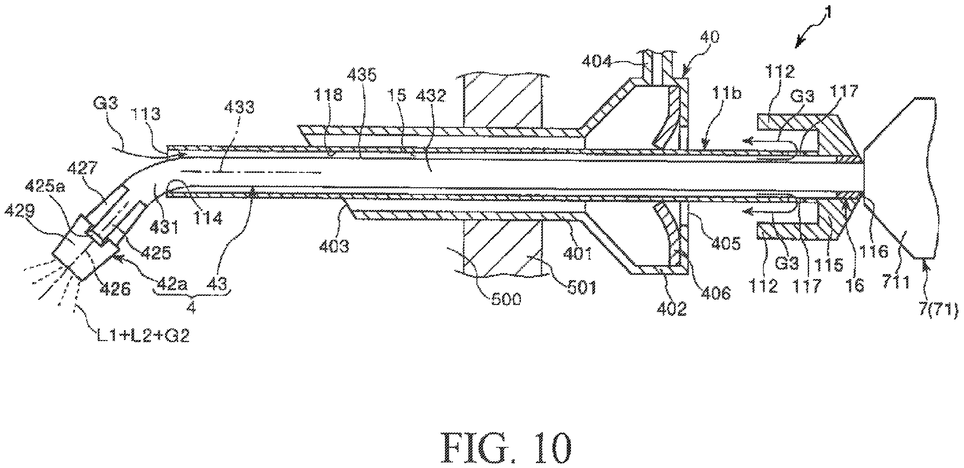

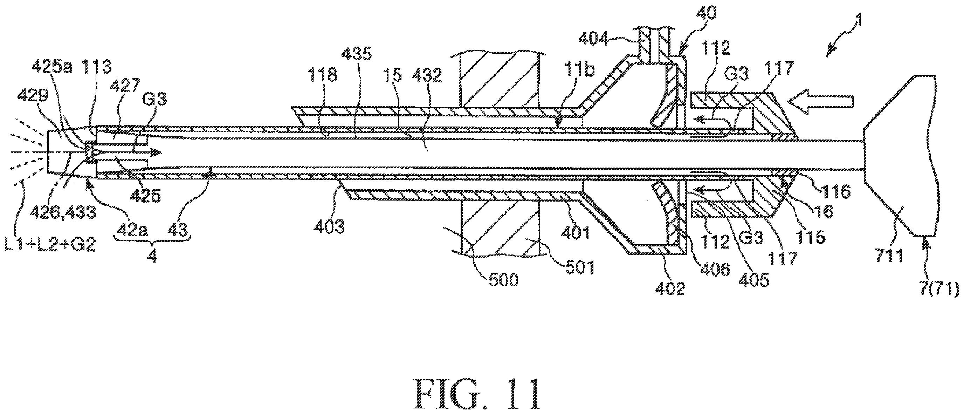

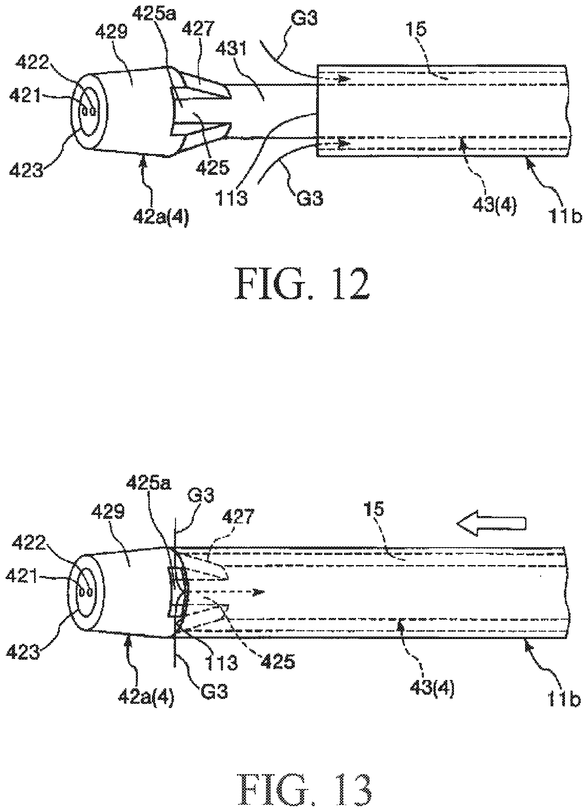

Figs. 10 and 11 are enlarged partial longitudinal views of an applicator (a fifth embodiment) of the present invention, Fig. 12 is a perspective view of the portion on the front end side of the applicator shown in Fig. 10, and Fig. 13 is a perspective view of the portion on the front end side of the applicator shown in Fig. 11.

Hereinafter, although a fifth embodiment of an applicator of the present invention will be described with reference to the drawings, the description will be focused on the points different from above-mentioned embodiment and the description of the same matter will be omitted.

The present embodiment is the same as the first embodiment except that a shape of a nozzle and a configuration of an outer tube differ.

Nozzle head 42a of nozzle 4 shown in each drawing does not have outer diameter constant portion 428, unlike nozzle head 42 of the first embodiment. On first taper portion 427 (the fitting portion) of nozzle head 42a, at least one groove 425 is formed along the direction of axis 426 of nozzle head 42a. Groove 425 is configured so that front end portion 425a thereof extends up to second taper portion 429. As shown in Figs. 11 and 13, even when axis 426 of nozzle head 42a coincides with axis 433 of nozzle main body 43, front end portion 425a of groove 425 prevents the overall front end opening portion 113 of sheath 11b from covering nozzle head 42a, and gap 15 communicates with the inner part of abdominal cavity 500 via front end portion 425a of groove 425. As a result, gas G3 within abdominal cavity 500 can reliably flow in gap 15 via front end portion 425a of groove 425.

As shown in Figs. 10 and 11, on proximal end opening portion 116 of sheath 11b, spacer 16 functioning as the above-mentioned positioning means is installed. For this reason, proximal end opening portion 116 is occluded. In this case, on the proximal end portion of sheath 11b, specifically, on the portion of the proximal end side further than the proximal end of trochal tube 40 with applicator 1 inserted therein, two side holes 117 communicating with gap 15 are formed. Gas G3 passed through gap 15 can reliably be discharged via the respective side holes 117. In this manner, the respective side holes 117 function as outflow ports through which gas G passed through gap 15 flows out.

On flange 115 of sheath 11b, two protruding pieces 112 protruding toward the front end direction are formed. These protruding pieces 112 are oppositely disposed via the axis of sheath 11b. When applicator 1 is gradually pushed in the front end direction in the state in which applicator 1 is inserted into trochal tube 40, each protruding piece 112 of sheath 11b respectively comes into contact with proximal end opening portion 405 of hub 402. As a result, the movement limitation of sheath 11b with respect to trochal tube 40 in the front end direction can be restricted, and thus it is possible to prevent the proximal end portion of sheath 11b from carelessly entering trochal tube 40. As a result, the proximal end portion of sheath 11b can reliably protrude from proximal end opening portion 405 of trochal tube 40, thereby grasping the protruded portion. Thus, sheath 11b can be subjected to movement operation. In this manner, each protruding piece 112 functions as the restriction means which restricts the movement limitation of sheath 11 with respect to trochal tube 40 in the front end direction.

Each protruding piece 112 is respectively disposed at a position of covering each side hole 117 of sheath 11b from the outer peripheral side thereof. As a result, when the proximal end portion of sheath 11b is grasped with fingers and is subjected to movement operation, it is possible to reliably prevent each side hole 117 from being blocked with fingers. Thus, gas G3 passed through gap 15 can further reliably be discharged via each side hole 117.

In addition, each protruding piece 112 may be omitted.

### Sixth Embodiment

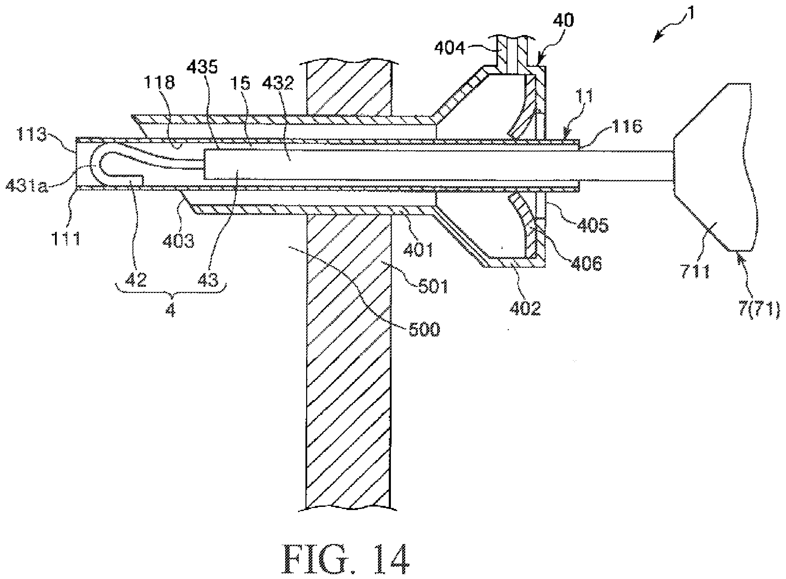

Figs. 14, 15 and 16 are enlarged partial longitudinal section views of an applicator (a sixth embodiment) of the present invention.

Hereinafter, although the sixth embodiment of an applicator of the present invention will be described with reference to the drawings, the description will be focused on the points different from the above-mentioned embodiment, and the description of the same matter will be omitted.

The present embodiment is the same as the first embodiment except that a shape of a curved section of a nozzle differs.

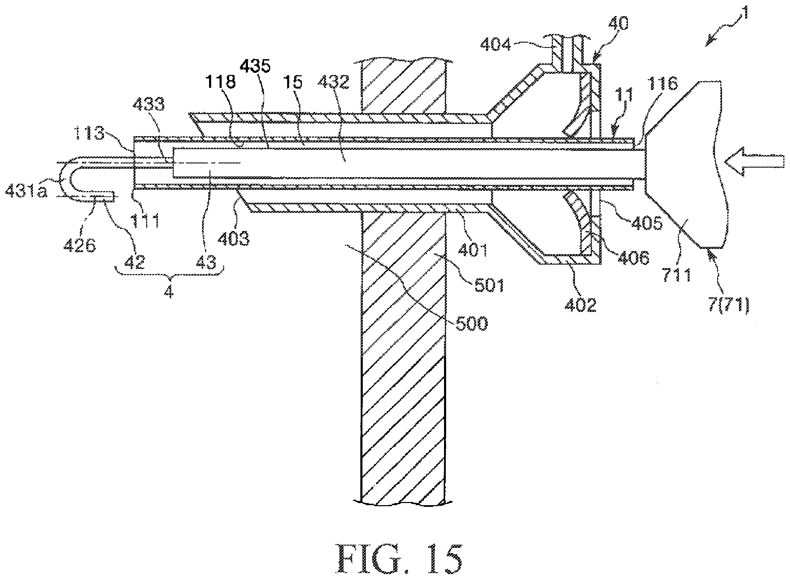

As shown in Fig. 15, curved section 431a of nozzle 4 is curved so that the shape thereof forms a U shape in the natural state. As a result, nozzle head 42 faces toward the proximal end side in the natural state. That is, axis 426 of nozzle head 42 is parallel to axis 433 of nozzle main body 43.

As shown in Fig. 14, in the state in which the overall curved section 431a is received in sheath 11, curved section 431a is restricted by inner peripheral surface 118 of sheath 11, whereby a degree of curve is slightly larger than during natural state.

When applicator main body 7 is pushed toward the front end direction from the state shown in Fig. 14, as shown in Fig. 15, the front end portion of nozzle 4 protrudes from front end opening portion 113 of sheath 11. At this time, curved section 431a enters the natural state as described above, and nozzle head 42 faces right behind.

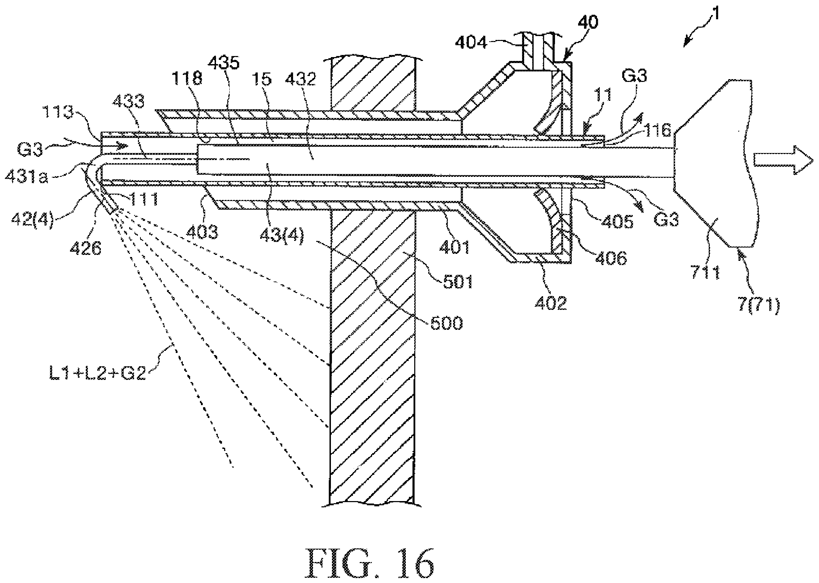

When applicator main body 7 is stretched toward the proximal end direction from the state shown in Fig. 15, as shown in Fig. 16, the proximal end portion of curved section 431a is received in sheath 11, so that nozzle head 42 (or curved section 431a) comes into contact with the outer edge portion 111 of front end opening portion 113 of sheath 11. When applicator main body 7 is further stretched toward the proximal end direction, nozzle head 42 is pressed toward the front end direction by edge portion 111, and the shape of curved section 431a is corrected so as to be opened. As a result, a degree of curve of curved section 431a becomes smaller than during natural state, and nozzle head 42 faces obliquely right downward in Fig. 16. When the mixture is expelled from nozzle head 42 in this state, the mixture is expelled toward the rear. As a result, the mixture can be applied to the portion on the rear side of abdominal wall 501.

In a case where the mixture wants to be expelled toward the front, by further stretching applicator main body 7 from the state shown in Fig. 16 toward the proximal end direction, curved section 431a is corrected by sheath 11 and is opened greater than the state shown in Fig. 16, whereby nozzle head 42 faces forward. In this state, the mixture can be expelled toward the front.

The mixture may be expelled from nozzle head 42 in the state shown in Fig. 15, that is, from the state in which nozzle head 42 faces directly behind. However, in this case, the mixture may be given to applicator 1 as well as the rear surface of abdominal wall 501.

It is desirable that edge portion 111 of front end opening portion 113 of sheath 11 be spherical.

In addition, in the present embodiment, nozzle head 42 is configured so that the portion in which the outer diameter changes along the longitudinal direction is omitted and it becomes constant. The outer diameter of nozzle head 42 is smaller than the inner diameter of sheath 11. As a result, regardless of the position of sheath 11 in the longitudinal direction of nozzle 4, gas G3 flows in gap 15 from the front end portion 113 of sheath 11 (see Fig. 16).

In addition, in the present embodiment, on the proximal end outer peripheral portion of sheath 11, flange 115 protruding from the proximal end outer peripheral portion of sheath 11 as in the first embodiment is omitted, but not limited thereto, flange 115 may be formed.

### Seventh Embodiment

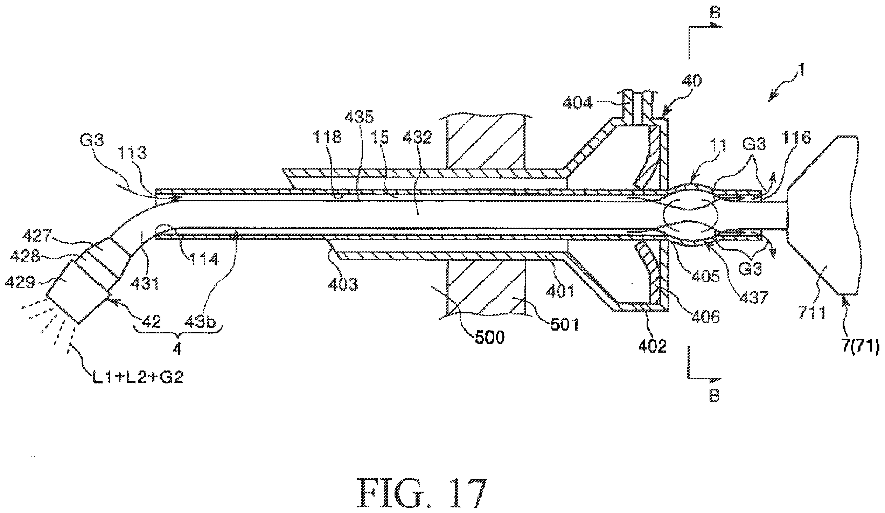

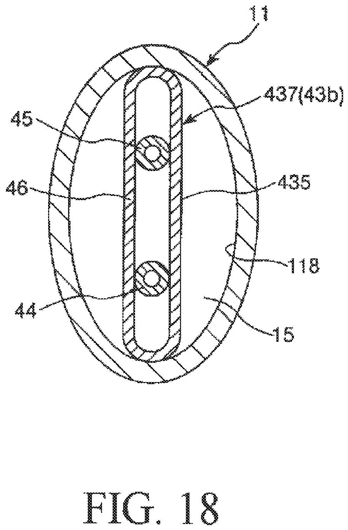

Fig. 17 is an enlarged partial longitudinal sectional view of an applicator (a seventh embodiment) of the present invention, and Fig. 18 is a sectional view taken from line B-B in Fig. 17.

Hereinafter, while the seventh embodiment of the applicator of the present invention will be described with reference to the drawings, the description will be focused on the points different from the above-mentioned embodiments and the description of the same matter will be omitted.

The present invention is the same as the first embodiment except that a partial shape of the nozzle main body of the nozzle differs.

As shown in Fig. 17, flat portion 437 is formed on the proximal end portion of nozzle main body 43b. Flat portion 437 is a portion which has deformed a part of the outer tube forming third flow path 46 so as to form a flat shape (see Fig. 18). The length of flat portion 437 in the vertical direction in Fig. 18 is larger than the inner diameter of sheath 11 in the natural state. As a result, outer peripheral surface 435 in flat portion 437 of nozzle main body 43b comes into close contact with inner peripheral surface 118 of sheath 11. When sheath 11 moves in the longitudinal direction in this state, inner peripheral surface 118 of sheath 11 slides on outer peripheral surface 435 of flat portion 437, and the frictional resistance is generated between inner peripheral surface 118 of sheath 11 and outer peripheral surface 435 of flat portion 437.

Flat portion 437 functions as a positioning means for performing the positioning of sheath 11 relative to the longitudinal direction of nozzle 4 in the stop position when sheath 11 moves and stops. As a result, slope angle θ of nozzle head 42 can be maintained, which can expel the mixture in this state.

In addition, as mentioned above, applicator 1 is used in the state of being inserted into trochal tube 40. When applicator 1 is pressed in the front end direction in this state, the outer peripheral portion of the portion where flat portion 437 of sheath 11 is situated comes into contact with the edge portion of proximal end opening portion 405 of hub 402 (see Fig. 17). As a result, the movement limitation of sheath 11 with respect to trochal tube 40 in the front end direction can be restricted, and thus it is possible to prevent the proximal end portion of sheath 11 from carelessly entering trochal tube 40, that is, to prevent the proximal end portion of sheath 11 from moving to the front end side further than proximal end opening portion 405 of trochal tube 40. As a result, the proximal end portion of sheath 11 can reliably protrude from proximal end opening portion 405 of trochal tube 40, thereby grasping the protruded portion. Thus, sheath 11 can be moved and operated. In this manner, flat portion 437 also functions as a restriction means for restricting the movement limitation of sheath 11 relative to trochal tube 40 in the front end direction.

While the shown embodiments of the applicator of the present invention have been described above, the present invention is not limited thereto, but the respective portions constituting the applicator can be replaced with an arbitrary constitution capable of exhibiting the same function. Furthermore, an arbitrary composition may be added.

The applicator of the present invention may be a combination of two or more arbitrary configurations (characteristics) among the respective embodiments.

The applicator may be used by being inserted into the abdominal cavity, but is not limited thereto, for example, the applicator may be used by being inserted into body cavities such as a thoracic cavity and a womb.

The applicator is used in a surgery in which gas is supplied from the trochal tube into the abdominal cavity to expand the abdominal cavity, but not limited thereto, for example, the applicator may be used in a surgery in which the abdominal wall is suspended to secure the size of the abdominal cavity, known as suspending method.

In a case where the trochal tubes are detained in a plurality of abdominal walls in the laparoscope, among the trochal tubes, for example, the gas may be supplied from the one trochal tube, and the supply of the gas from other trochal tubes may stop.

The spacer is installed on one place in the longitudinal direction of the sheath in the first to third embodiments, but not limited thereto, the spacer may be installed on a plurality of places in the longitudinal direction of the sheath.

In the first to third embodiments, the spacer is configured so that the outer peripheral surface of the spacer is fixed to the inner peripheral surface of the sheath and the inner peripheral surface of the spacer is not fixed to but is in contact with the outer peripheral surface of the nozzle main body, but is not limited thereto, for example, the inner peripheral surface of the spacer is fixed to the outer peripheral surface of the nozzle main body and the outer peripheral surface of the spacer may not be fixed to but may be in contact with the inner peripheral surface of the sheath.

The protruding portion as the positioning means is formed on the inner peripheral surface of the sheath in the fourth embodiment, but is not limited thereto, for example, the protruding portion may be formed on the outer peripheral surface of the nozzle main body.

Furthermore, the applicator expel liquid together the gas, but may apply powder.

### Industrial Applicability

The applicator of the present invention is an applicator which is used by being inserted into the living body that includes a nozzle which has a longer nozzle main body, and a nozzle head, which is provided on a front end side of the nozzle main body and through which liquid together with gas is expelled, with a curved section, which is curved or bent and has flexibility, being formed on the front end portion of the nozzle main body; and an outer tube through which the nozzle main body is inserted so as to be movable along a longitudinal direction thereof and an angle of a curve of the curved section changes by inserting the curved section, thereby adjusting a direction of the nozzle head relative to an axis of the nozzle main body, and wherein a gap is formed along the longitudinal direction between the outer tube and the nozzle, and the gap functions as a discharge path for discharging the gas within the body cavity via the gap to the outside of the body, when a body pressure within a body cavity rises. For this reason, by moving the outer tube along the longitudinal direction to suitably adjust the direction of the nozzle head relative to the axis of the nozzle main body, it is possible to expel the liquid from the nozzle head toward a plurality of places (e.g., organs etc.) within the body cavity, while changing the direction thereof. Thus, the applicator can easily and reliably apply liquid or powder within the body cavity over a wide range. Furthermore, due to the gas expelled from the nozzle head, the body pressure within the body cavity rises and the body cavity is expanded. For example, when the gas continues to be expelled, the body pressure within the body cavity excessively rises, whereby the body cavity tries to further expand. However, the gas within the body cavity flows in the gap. The flowed-in gas flows down in the gap and is discharged to the outside. As a result, it is possible to suppress or prevent an excessive rise in body pressure within the body cavity, which can prevent the body cavity from trying to expand. In addition, in cases where the curved section is curved to a degree that the nozzle head faces the proximal end side in the natural state of not giving an external force, for example, when the applicator of the present invention is used in the laparoscopic surgery, the liquid or the powder can be applied to the rear surface (the rear portion) of the abdominal wall. Thus, the applicator of the present invention has industrial applicability.

## Claims

1. An applicator (1) adapted for being inserted into a living body including:
a nozzle (4) which has a nozzle main body (43), and a nozzle head (42), which is provided on a front end side of the nozzle main body (43) and which is suitable to expel liquid or powder together with gas, with a curved section (431), which is curved or bent and has flexibility, being formed on the front end portion of the nozzle main body (43); and
an outer tube (11) through which the nozzle main body (43) is inserted so as to be movable along a longitudinal direction thereof and a shape of the curved section (431) is corrected by inserting the curved section (431), thereby adjusting a direction of the nozzle head (42) relative to an axis (433) of the nozzle main body (43),
wherein a gap (15) is formed along the longitudinal direction between the outer tube (11) and the nozzle (4), and
wherein the gap (15) is suitable to function as a discharge path for discharging the gas within the body cavity via the gap (15) to the outside of the body when the body pressure within the body cavity rises,
wherein the applicator (1) further includes
a positioning means (16, 16a) for positioning the outer tube (11) with respect to the longitudinal direction of the nozzle (4),
**characterized in that**
the positioning means (16, 16a) includes an elastic body which is fixed with respect to one surface of an outer peripheral surface of the nozzle (4) main body and an inner peripheral surface of the outer tube (11) in the middle of the gap (15) and comes into contact with the other surface thereof.

2. The applicator (1) of Claim 1, wherein
the outer tube (11) has a front end opening portion (403) in which a front end thereof is opened to communicate with the gap (15), and
wherein the front end opening portion (403) is suitable to function as an inlet through which the gas within the body cavity flows in the gap (15).

3. An applicator (1) of Claim 1, wherein
the elastic body seals the gap (15) therein, and wherein, in the outer tube (11), a side hole communicating with the gap (15) is formed on a portion of a proximal end side further than a portion where the elastic body is installed.

4. An applicator (1) of Claim 1, wherein
the nozzle head (42) has a fitting portion (711) which is fitted to the front end opening portion (403) of the outer tube (11) when an axis (426) of the nozzle head (42) coincides with an axis (433) of the nozzle main body (433)..

5. An applicator (1) of Claim 4, wherein
at least one groove is formed in the fitting portion (711) along an axial direction of the nozzle head (42), and wherein, when the axis (426) of the nozzle head (42) coincides with the axis (433) of the nozzle main body (43), the gas within the body cavity flows in the gap (15) via the groove.

6. An applicator (1) of Claim 4, wherein
the nozzle main body (43) has a liquid flow path, which is connected to a liquid supply means (2) for supplying the liquid and through which the liquid from the liquid supply means (2) passes, and a gas flow path which is connected to a gas supply means for supplying the gas and through which the gas from the gas supply means passes.

7. An applicator (1) adapted for being inserted into a living body including:
a nozzle (4) which has a nozzle main body (43), and a nozzle head (42), which is provided on a front end side of the nozzle main body (43) and which is suitable to expel liquid or powder together with gas, with a curved section (431), which is curved or bent and has flexibility, being formed on the front end portion of the nozzle main body (43); and
an outer tube (11) through which the nozzle main body (43) is inserted so as to be movable along a longitudinal direction thereof and a shape of the curved section (431) is corrected by inserting the curved section (431), thereby adjusting a direction of the nozzle head (42) relative to an axis (433) of the nozzle main body (43),
wherein a gap (15) is formed along the longitudinal direction between the outer tube (11) and the nozzle (4), and
wherein the gap (15) is suitable to function as a discharge path for discharging the gas within the body cavity via the gap (15) to the outside of the body when the body pressure within the body cavity rises,
wherein the applicator (1) further includes
a positioning means for positioning the outer tube (11) with respect to the longitudinal direction of the nozzle (4),
**characterized in that**
the positioning means includes a protruding portion (119) which protrudes from one surface of the outer peripheral surface of the nozzle main body (43) and the inner peripheral surface of the outer tube (11) in the middle of the gap (15) and comes in contact with the other surface thereof.

## Patentansprüche

1. Applikator (1), der zum Einsetzen in einen lebenden Körper eingerichtet ist, umfassend:
eine Düse (4), die einen Düsenhauptkörper (43) und einen Düsenkopf (42) aufweist, der an einer vorderen Stirnseite des Düsenhauptkörpers (43) vorgesehen ist und geeignet ist, Flüssigkeit oder Pulver zusammen mit Gas auszustoßen, wobei ein gekrümmter Abschnitt (431), der gekrümmt oder gebogen ist und eine Flexibilität aufweist, an dem vorderen Endabschnitt des Düsenhauptkörpers (43) ausgebildet ist; und
ein Außenrohr (11), durch das der Düsenhauptkörper (43) eingeführt ist, so dass er entlang einer Längsrichtung davon bewegbar ist, und eine Form des gekrümmten Abschnitts (431) durch Einsetzen des gekrümmten Abschnitts (431) korrigiert wird, wodurch eine Richtung des Düsenkopfes (42) relativ zu einer Achse (433) des Düsenhauptkörpers (43) eingestellt wird,
wobei ein Spalt (15) entlang der Längsrichtung zwischen dem Außenrohr (11) und der Düse (4) ausgebildet ist, und
wobei der Spalt (15) geeignet ist, um als ein Ableitungsweg zum Ableiten des Gases innerhalb der Körperhöhle über den Spalt (15) zur Außenseite zu wirken, wenn der Körperdruck innerhalb der Körperhöhle ansteigt,
wobei der Applikator (1) ferner umfasst
eine Positioniereinrichtung (16, 16a) zum Positionieren des Außenrohres (11) in Bezug auf die Längsrichtung der Düse (4),
**dadurch gekennzeichnet, dass**
die Positioniereinrichtung (16, 16a) einen elastischen Körper aufweist, der bezüglich einer Fläche einer äußeren Umfangsfläche des Hauptkörpers der Düse (4) und einer inneren Umfangsfläche des Außenrohres (11) in der Mitte des Spaltes (15) befestigt ist und mit der anderen Fläche davon in Kontakt kommt.

2. Applikator (1) nach Anspruch 1, wobei
das Außenrohr (11) einen vorderen Endöffnungsabschnitt (403) aufweist, in dem ein vorderes Ende davon geöffnet ist, um mit dem Spalt (15) zu kommunizieren, und
wobei der vordere Endöffnungsabschnitt (403) geeignet ist, als ein Einlass zu fungieren, durch den das Gas innerhalb des Körperhohlraums in den Spalt (15) strömt.

3. Applikator (1) nach Anspruch 1, wobei
der elastische Körper den Spalt (15) darin abdichtet, und
wobei in dem Außenrohr (11) ein Seitenloch, das mit dem Spalt (15) in Verbindung steht, auf einem Abschnitt einer proximalen Endseite ausgebildet ist, der entfernter als ein Abschnitt ist, an dem der elastische Körper installiert ist.

4. Applikator (1) nach Anspruch 1, wobei
der Düsenkopf (42) einen Passabschnitt (711) aufweist, der an dem vorderen Endöffnungsabschnitt (403) des Außenrohres (11) angebracht ist, wenn eine Achse (426) des Düsenkopfes (42) mit einer Achse (433) des Düsenhauptkörpers (43) zusammenfällt.

5. Applikator (1) nach Anspruch 4, wobei
mindestens eine Nut in dem Passabschnitt (711) entlang einer axialen Richtung des Düsenkopfes (42) ausgebildet ist, und wobei, wenn die Achse (426) des Düsenkopfes (42) mit der Achse (433) des Düsenhauptkörpers (43) zusammenfällt, das Gas innerhalb des Körperhohlraums über die Nut in den Spalt (15) strömt.

6. Applikator (1) nach Anspruch 4, wobei
der Düsenhauptkörper (43) einen Flüssigkeitsströmungsweg aufweist, der mit einer Flüssigkeitszuführungseinrichtung (2) zum Zuführen der Flüssigkeit verbunden ist und durch den die Flüssigkeit aus der Flüssigkeitszuführungseinrichtung (2) hindurchtritt, und
einen Gasströmungsweg, der mit einer Gaszuführungseinrichtung zum Zuführen des Gases verbunden ist, und durch den das Gas von der Gaszuführungseinrichtung hindurchtritt.

7. Applikator (1), der zum Einsetzen in einen lebenden Körper eingerichtet ist, umfassend:
eine Düse (4), die einen Düsenhauptkörper (43) und einen Düsenkopf (42) aufweist, der an einer vorderen Stirnseite des Düsenhauptkörpers (43) vorgesehen ist und geeignet ist, Flüssigkeit oder Pulver zusammen mit Gas auszustoßen, wobei ein gekrümmter Abschnitt (431), der gekrümmt oder gebogen ist und eine Flexibilität aufweist, an dem vorderen Endabschnitt des Düsenhauptkörpers (43) ausgebildet ist; und
ein Außenrohr (11), durch das der Düsenhauptkörper (43) eingeführt ist, so dass er entlang einer Längsrichtung davon bewegbar ist, und eine Form des gekrümmten Abschnitts (431) durch Einsetzen des gekrümmten Abschnitts (431) korrigiert wird, wodurch eine Richtung des Düsenkopfes (42) relativ zu einer Achse (433) des Düsenhauptkörpers (43) eingestellt wird,
wobei ein Spalt (15) entlang der Längsrichtung zwischen dem Außenrohr (11) und der Düse (4) ausgebildet ist, und
wobei der Spalt (15) geeignet ist, um als ein Ableitungsweg zum Ableiten des Gases innerhalb der Körperhöhle über den Spalt (15) zur Außenseite zu wirken, wenn der Körperdruck innerhalb der Körperhöhle ansteigt,
wobei der Applikator (1) ferner umfasst
eine Positioniereinrichtung zum Positionieren des Außenrohres (11) in Bezug auf die Längsrichtung der Düse (4),
**dadurch gekennzeichnet, dass**
die Positioniereinrichtung einen vorstehenden Abschnitt (119) aufweist, der von einer Fläche der äußeren Umfangsfläche des Düsenhauptkörpers (43) und der inneren Umfangsfläche des Außenrohrs (11) in der Mitte des Spalts (15) vorsteht und mit der anderen Fläche davon in Kontakt kommt.

## Revendications

1. Applicateur (1) adapté pour être inséré dans un corps vivant, comprenant :
une buse (4) qui comporte un corps principal de buse (43), et une tête de buse (42), qui est placée sur un côté d'extrémité avant du corps principal de buse (43) et qui est adaptée pour expulser un liquide ou une poudre en même temps que du gaz, une section courbée (431), qui est courbée ou fléchie et présente une flexibilité, étant formée sur la partie d'extrémité avant du corps principal de buse (43) ; et
un tube extérieur (11) à travers lequel est inséré le corps principal de buse (43) de manière à être mobile dans la direction longitudinale de celui-ci et une forme de la section courbée (431) est corrigée par l'insertion de la section courbée (431), en ajustant ainsi une direction de la tête de buse (42) par rapport à un axe (433) du corps principal de buse (43),
où un espace (15) est formé dans la direction longitudinale entre le tube extérieur (11) et la buse (4), et
où l'espace (15) est adapté pour servir de passage d'évacuation pour décharger le gaz présent à l'intérieur de la cavité corporelle par l'intermédiaire de l'espace (15) vers l'extérieur du corps lorsque la pression du corps à l'intérieur de la cavité corporelle augmente,
où l'applicateur (1) comprend en outre
un moyen de positionnement (16, 16a) pour positionner le tube extérieur (11) par rapport à la direction longitudinale de la buse (4),
**caractérisé en ce que**
le moyen de positionnement (16, 16a) comprend un corps élastique qui est fixé par rapport à une surface d'une surface périphérique extérieure du corps principal de la buse (4) et d'une surface périphérique intérieure du tube extérieur (11) au milieu de l'espace (15) et vient en contact avec l'autre surface de celui-ci.

2. Applicateur (1) selon la revendication 1, dans lequel
le tube extérieur (11) comporte une partie d'ouverture d'extrémité avant (403) dans laquelle son extrémité avant est ouverte pour communiquer avec l'espace (15), et
où la partie d'ouverture d'extrémité avant (403) est adaptée pour servir d'entrée à travers laquelle le gaz présent à l'intérieur de la cavité corporelle s'écoule dans l'espace (15).

3. Applicateur (1) selon la revendication 1, dans lequel
le corps élastique ferme hermétiquement l'espace (15), et où, dans le tube extérieur (11), un trou latéral communiquant avec l'espace (15) est formé sur une partie d'un côté d'extrémité proximale plus éloignée qu'une partie où le corps élastique est installé.

4. Applicateur (1) selon la revendication 1, dans lequel
la tête de buse (42) comporte une partie d'ajustage (711) qui est assemblée à la partie d'ouverture d'extrémité avant (403) du tube extérieur (11) quand un axe (426) de la tête de buse (42) coïncide avec un axe (433) du corps principal de buse (43).

5. Applicateur (1) selon la revendication 4, dans lequel
au moins une rainure est formée dans la partie d'ajustage (711) le long d'une direction axiale de la tête de buse (42), et où, quand l'axe (426) de la tête de buse (42) coïncide avec l'axe (433) du corps principal de buse (43), le gaz présent à l'intérieur de la cavité corporelle s'écoule dans l'espace (15) par l'intermédiaire de la rainure.

6. Applicateur (1) selon la revendication 4, dans lequel
le corps principal de buse (43) comporte un trajet d'écoulement de liquide, qui est raccordé à un moyen de distribution de liquide (2) pour distribuer le liquide et par lequel passe le liquide venant du moyen de distribution de liquide (2), et un trajet d'écoulement de gaz qui est raccordé à un moyen de distribution de gaz pour distribuer le gaz et par lequel passe le gaz venant du moyen de distribution de gaz.

7. Applicateur (1) adapté pour être inséré dans un corps vivant, comprenant :
une buse (4) qui comporte un corps principal de buse (43), et une tête de buse (42), qui est placée sur un côté d'extrémité avant du corps principal de buse (43) et qui est adaptée pour expulser un liquide ou une poudre en même temps que du gaz, une section courbée (431), qui est courbée ou fléchie et présente une flexibilité, étant formée sur la partie d'extrémité avant du corps principal de buse (43) ; et
un tube extérieur (11) à travers lequel est inséré le corps principal de buse (43) de manière à être mobile dans la direction longitudinale de celui-ci et une forme de la section courbée (431) est corrigée par l'insertion de la section courbée (431), en ajustant ainsi une direction de la tête de buse (42) par rapport à un axe (433) du corps principal de buse (43),
où un espace (15) est formé dans la direction longitudinale entre le tube extérieur (11) et la buse (4), et
où l'espace (15) est adapté pour servir de passage d'évacuation pour décharger le gaz présent à l'intérieur de la cavité corporelle par l'intermédiaire de l'espace (15) à l'extérieur du corps lorsque la pression du corps à l'intérieur de la cavité corporelle augmente,
où l'applicateur (1) comprend en outre
un moyen de positionnement pour positionner le tube extérieur (11) par rapport à la direction longitudinale de la buse (4),
**caractérisé en ce que**
le moyen de positionnement comprend une partie saillante (119) qui fait saillie depuis une surface de la surface périphérique extérieure du corps principal de buse (43) et de la surface périphérique intérieure du tube extérieur (11) au milieu de l'espace (15) et qui vient en contact avec l'autre surface de celui-ci.
